# EUROPEAN PATENT SPECIFICATION

(11) **EP 0 309 471 B1**
(45) Date of publication and mention of the grant of the patent: **14.08.1996**
(21) Application number: 87903961.8
(22) Date of filing: 02.06.1987
(51) Int. Cl.: A61B 6/00

(54) **CATHETER**
KATHETER-SONDE
SONDE

(30) Priority: 02.06.1986 US 869597
(43) Date of publication of application: 05.04.1989
(60) Divisional application: 93117632.5
(73) Proprietor: TARGET THERAPEUTICS, INC., Fremont, CA 94537-5120 (US)
(72) Inventor: ENGELSON, Erik, T., Palo Alto, California 94306 (US); VEGH, Gabriel, B., Danville, CA 94526 (US)
(74) Representative: Senior, Alan Murray
(86) International application number: PCT/US87/01281
(87) International publication number: WO 87/07493

(56) References cited:
- EP-A- 0 102 685
- US-A- 3 406 685
- US-A- 4 239 042
- US-A- 4 385 635
- US-A- 4 417 886
- US-A- 4 545 390
- US-A- 4 665 746

## Description

The present invention relates to a catheter device for accessing a tissue target site via a small-lumen tortuous path within a target tissue.

Catheters are being used increasingly as a means for delivering diagnostic or therapeutic agents to internal target sites that can be accessed through the circulatory system. For example, in angiography, catheters are designed to deliver a radio-opaque agent to a target site within a blood vessel, to allow radiographic viewing of the vessel and blood flow characteristics near the release site. For the treatment of localized disease, such as solid tumors, catheters allow a therapeutic agent to be delivered to the target site at a relatively high concentration, with minimum overall side effects. Methods for producing localized vaso-occlusion in target tissue regions, by catheter injection of a vaso-occlusive agent, have also been described in co-owned US-A-4708718.

Often the target site which one wishes to access by catheter is buried within a soft tissue, such as brain or liver, and can be reached only by a tortuous route through small vessels or ducts, --typically less than about 3 mm lumen diameter-- in the tissue. The difficulty in accessing such regions is that the catheter must be quite flexible, in order to follow the tortuous path into the tissue, and at the same time, stiff enough to allow the distal end of the catheter to be manipulated from an external access site, which may be as much as a meter or more from the tissue site.

Heretofore, two general methods for accessing such tortuous-path regions have been devised. The first method employs a highly flexible catheter having an inflatable, but pre-punctured balloon at its distal end. In use, the balloon is partially inflated, and carried by blood flow into the target site. The balloon is continually inflated during placement to replenish fluid leaking from the balloon. A major limitation of this method is that the catheter will travel in the path of highest blood flow rate, so many target sites with low blood flow rates cannot be accessed.

In the second prior art method, a torqueable guide wire having a distal bend is guided, by alternately rotating and advancing the wire, to the target site. With the wire in place, a thin-walled catheter is then advanced along the wire until the distal catheter end is positioned at the target site. Once the catheter is advanced, the guide wire may be withdrawn to allow fluid delivery through the catheter. An important advantage of this method is the ability to control the location of the catheter along a vascular pathway. However, prior art catheter/guide wire devices have limited ability to reach target sites along small-lumen tortuous paths, such as within the vascular system leading to deep brain sites, making these sites largely inaccessible by prior art catheter devices.

Considering first the limitations of prior art guide wires, these are typically formed of flexible, torqueable filament material, such as stainless steel, and have preferred diameters of between about 0.2 to 1 mm (8-40 thousandths of an inch - "mils"). The distal end of the wire may be provided with a bent tip which can be oriented, by means of guide structure at the proximal end, to guide the wire along a selected vascular path. Ideally, torque transmission should be controlled, such that a selected wire rotation at the wire's proximal end produces a corresponding rotation of the distal end. Because of their greater flexibility, smaller diameter wires, e.g., having diameters of between about 0.2 to 0.45 mm (8-18 mils), may be required for accessing small-vessel and/or tortuous-path regions. However, if the wire is too thin along its entire length, it may be difficult to transmit torque in a controlled manner along the entire wire length, and the wire may buckle, preventing withdrawal.

More recently, guide wires which have variable-thickness steps along the wire length have been proposed. Wires of this type have the advantage that the proximal end region; where greater torsional strength is required, have relatively large diameters, e.g., between about 0.5 to 1 mm (20-40 mils), and the distal end region, where greater flexibility is required, have relatively small diameters. Typically, a wire of this type will have two or three different-diameter segments extending collectively over an approximately 25-60 cm distal portion of the wire, and a short (typically, 1-3 cm) tapered transition zone across each step. The tapered zones are typically formed by centerless grinding in which the wire is placed between two counter-rotating grinding wheels whose confronting grinding surfaces are angled slightly to produce the desired taper over the width of the wheels.

Despite its advantage of combining high torque strength in the proximal region with good distal-region flexibility, the variable-step taper wire just described has several disadvantages. One of these is the tendency of the wire to bend sharply in a step (transition) zone when a sharp turn in the vessel path is encountered, due to the differential bending modulus at the transition zone. If the catheter on the wire has already been advanced past the point of the bend, the catheter may deform at the wire bend, making further catheter advance along the wire difficult or impossible. If the wire is sharply bent at two spaced transition zones, it may be impossible to advance or withdraw the catheter over the wire. Further, torqueability in the wire is reduced at the region of a sharp bend, since torque tends to be transmitted through the angle of the bend, rather than along the axis of the wire.

Additionally, the transition zones in the discontinuous-taper wire are potential weak spots for torsional breaks, due to the high torsional differential across these zones. (The torsional force is related to the fourth power of the wire thickness). At best, torque transmission across the short, tapered step zones is inefficient.

The problems of advancing a catheter along a guide wire in a small-lumen, tortuous tissue pathway are also due to limitations in prior art catheter construction. If the catheter is relatively rigid, it cannot track over the final distal portion of the wire in the tortuous path region, because catheter advancement buckles the wire in a narrow turn, or because catheter advancement pulls the wire out of the distal vessels. On the other band, catheters having more flexible shafts, such as those used in balloon flow-directed devices, lack the column strength in the catheter's proximal section to be advanced over the guide wire without buckling. US-A-4385635 discloses an angiographic catheter having a soft terminal end, to avoid injury to an artery or vessel, a main reinforced length of polyamide jacketed by polyurethane, and a tapered zone containing a progressively thinner polyamide inner layer. This polyamide inner layer is intended to provide high bursting strength, rigidity, torque control and sufficient compressive strength as to allow the catheter to be advanced to the desired target. Whilst the catheter has three different sections of different stiffnesses, the catheter is not intended for use with a guidewire, nor is it for use in accessing subselective vessel paths.

EP-A-0102685 discloses a catheter for use with a guidewire. The catheter is essentially a two-segment catheter and has a relatively stiff proximal segment and a relatively flexible distal segment. As a consequence of its method of manufacture the catheter also includes a "slightly enlarged non-drawn portion.. to produce additional strength" between its proximal and distal segments. It appears that this non-drawn portion will be stiffer than the proximal catheter segment at least.

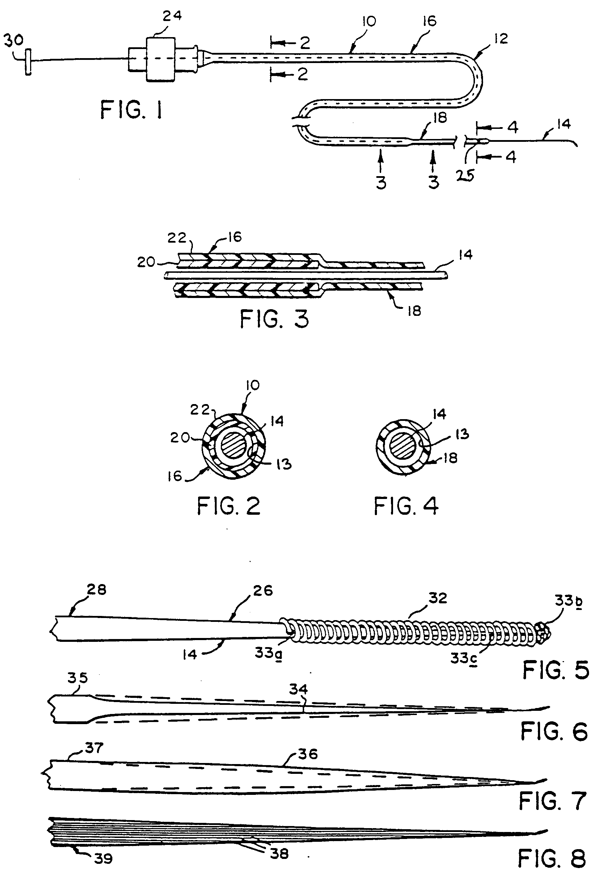

DE-B-21 40 755 discloses various different embodiments of plastic tubes having different sections of differing flexibilities. By utilising at least two extruders, each for extruding a different material type, both extruding through a common die a tube of unitary construction may be produced without a discrete join between two separately produced tubes of differing flexibilities. The Fig. 5 device is an intraveneous cannula which becomes more flexible towards its distal end. This cannula is, in use, mounted on a longer hypodermic needle for insertion into a patient. The tip section is made of soft material so as to reduce injury to the vein into which it is inserted. The proximal portion of the cannula is made of a stiff material so as to facilitate insertion of the needle arrangement into the cannular.

According to the present invention there is provided a catheter for use with a guidewire, the catheter being guidable from an external body access site to and into a soft internal brain or liver tissue, the catheter comprising:
an elongate tubular member having proximal and distal ends and an inner lumen extending between the proximal and distal ends, said member being composed of:
a relatively stiff proximal segment dimensioned to track the guidewire from the access site to a region adjacent the internal tissue;
a relatively flexible distal segment constructed and dimensioned to track the guidewire from said region to a target site within the internal tissue along the entire length of a tortuous path within the internal tissue of at least about 5 cm, through vessels of less than about 3 mm lumen inner diameter and around a plurality of sharp bends some of which may be of 90° or more, by means of an axially directed force applied to the distal segment through the proximal segment, the distal segment having both a proximal portion and a distal portion and said proximal portion including one or more intermediate segments having lesser flexibility than the distal portion of the distal segment and greater flexibility than the proximal segment.

The catheter is designed to be threaded over the wire, with such placed within the vessel pathway. In the preferred embodiment, the wire is provided with a flexible coil extending along and encasing the tapered region of the wire, and the clearance between the coil-encased portion of the wire and the inner diameter of the catheter distal segment is between about 0.05 to 0.12 mm (2-5 mils).

In a preferred embodiment, the guide wire is a flexible, torqueable wire having a length between about 50-300 cm from proximal to distal end, a maximum wire diameter of between about 0.2 to 1 mm (8-40 mils), and a region at least 15 cm in length having a continuously diminishing diameter on progressing toward the distal end of the wire. Also in the preferred embodiment, the wire is provided with a flexible coil extending along and encasing at least a major portion of the tapered region of the wire, and the clearance between the coil-encased portion of the wire and the inner diameter of the catheter distal segment is between about 0.05 to 0.12 mm (2-5 mils).

The catheter preferably has a coaxial tube construction in which the distal end portion of the proximal segment is formed of inner and outer coaxial tubes, one of which is relatively stiff and one of which is relatively flexible, and the distal segment is a distal extension of the relatively flexible tube. The relatively stiff tube may be formed of polypropylene or high-density polyethylene, and the relatively flexible tube, of low-density polyethylene or silicone.

An embodiment of catheter in accordance with the present invention will now be described, by way of example, with reference to the accompanying drawings, in which:
Figure 1 shows an embodiment of a basic catheter without the modification(s) necessary to make it in accordance with the present invention;
Figure 2 is an enlarged cross-sectional view taken along section line 2-2 in Figure 1;
Figure 3 is an enlarged side sectional view taken along line 3-3 in Figure 1;
Figure 4 is an enlarged cross-sectional view taken along line 4-4 in Figure 1;
Figures 5-7 each show, in exaggerated width dimension, the continuously variable distal end region of a tapered wire whose taper is substantially linear (Figure 5), concave (Figure 6) and convex (Figure 7);
Figure 8 is an enlarged sectional view of an end region of a composite tapered guide wire suitable for use with a catheter in accordance with the present invention;
Figure 9 illustrates a portion of a tortuous path in a soft tissue, and a method of guiding a catheter in accordance with the present invention along this path;
Figure 10 is a sectional view of a region of the small-diameter vessel path show in Figure 9, illustrating how bends in the path can produce sharp bending in a variable-step type guide wire, and buckling in a catheter which is threaded over the wire;
Figure 11 is an enlarged cross-sectional view taken generally along line 11-11 in Figure 10, showing distortion of the catheter at a wire bend;
Figure 12 is similar to Figure 10, but shows the bending which occurs in the continuously variable region of a guide wire in use with a catheter according to the present invention, and the condition of a catheter which is threaded over the wire; and
Figure 13 is an enlarged cross-sectional view taken generally along line 13-13 in Figure 12, showing distortion of the catheter at a wire bend.

### Detailed Description of the Invention

### I. Catheter Device

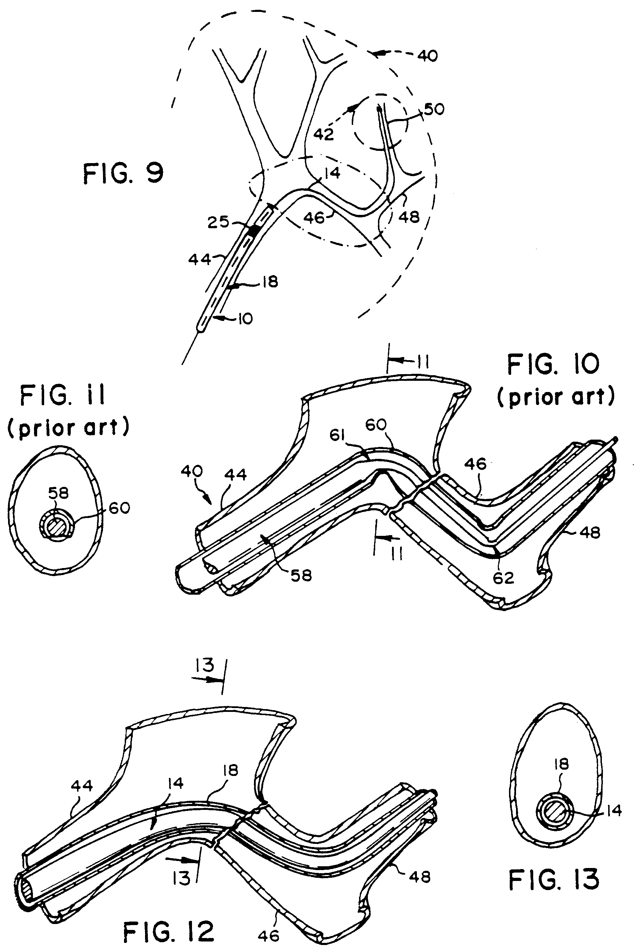

Figure 1 shows an embodiment of catheter 10 device without the modification(s) necessary (described below)to make it in accordance with the present invention. The device includes a catheter 12 which will be described particularly with respect to Figures 2-4 below, and a guide wire 14, whose construction is detailed with respect to Figures 5-8. The device is designed for accessing a target site which can be reached only along a small-lumen tortuous vessel path within a target tissue, as will be discussed with reference to Figures 9-13.

### A. Catheter

With reference first to Figures. 2-4, catheter 12 has a relatively stiff proximal segment 16 which makes up between about 70%-95% of the total tube length, and a relatively flexible distal segment 18 which makes up the remaining approximately 5%-30% of the tube length. With reference particularly to Figure 2, the proximal segment is composed of inner and outer coaxial tubes 20, 22 which are tight-fitting with respect to each other. The stiffness in the proximal segment is provided predominantly by tube 20. The inner, stiffer tube which here defines the lumen 13 of the catheter is preferably polypropylene or high-density polyethylene tubing having a final wall thickness (in the assembled catheter) of between about 0.05 to 0.1 mm (2-4 mils). The outer, more flexible tube is preferably low-density polyethylene or silicone tubing, also having a preferred wall thickness of between about 0.05 to 0.1 mm (2-4 mils). As defined herein, high- and low-density polyethylene have the usual trade meaning which is applied to the density grade of polyethylenes which are commonly used in extrusion.

It will be recognized that other tubing materials whose wall thickness can be adjusted to give comparable tubing flexibilities will be suitable, with the constraint that that the combined wall thickness of the two tubes should be less than about 0.25 mm (10 mils), and that the number of tubing layers of constant or varying flexibility forming the segments, or portions thereof, can be varied to achieve desired flexibility properties in the tube. It is also recognized that since the outer tube makes a relatively minor contribution to the total stiffness of the proximal segment, the wall thickness of the outer tube will generally be less than about 0.12 mm (5 mils).

With reference to Figure 2, the inner diameter of the proximal segment is dimensioned, with respect to the guide wire, to provide sufficient wire clearance to allow the catheter to be moved easily over the wire in an axial direction, during catheter placement at the target site. The guide wire itself must have a relatively small diameter, to permit its guided movement along a tortuous path in a target tissue. The construction of the guide wire will be detailed below. It is noted here only that the guide wire may have a continuously varying taper along its distal end region, and that this tapered region (or distal portion thereof) may be encased in a flexible coil which imparts a substantially constant outer diameter to the tapered region or portion of the wire.

The inner diameter of the catheter, particularly in its distal segment, is preferably between 0.05 to 0.12 mm (2-5 mils) larger than the guidewire for which the catheter is designed. Where the wire has a tapered distal region, the distal end of the catheter may itself be tapered to preserve this spacing within certain limits of axial movement of the wire within the catheter. Similarly, where the guide wire is untapered, or has a tapered portion which is covered by a substantially constant-diameter flexible coil (as shown in the figure), the distal end segment of the catheter would have a substantially constant inner diameter. Thus, for example, a catheter designed for use with constant-diameter guide wire whose outer diameter is 0.45 mm (18 mil) has a preferred inner diameter of 0.5 to 0.6 mm (20-25 mils), and more preferably 0.52 to 0.54 mm (21-22 mils). The preferred 0.05 to 0.12 mm (2-5 mil) total clearance between the wire and inner wall of the segment reduces the tendency of the segment to buckle under compressional strain, since the wire provides column support against tube bending and crimping. Although the inner diameter of the stiffer proximal segment may also be 0.05 to 0.12 mm (2-5 mils) larger than the diameter of the guidewire, and thus provide column support in the distal segment, a larger diameter proximal segment would provide for greater fluid flow through the catheter, in delivering fluid to the target site. In this arrangement, the proximal and distal segments would meet at a step, which would preferably be tapered to provide improved fluid flow.

The optimal length of the proximal segment will vary according to the distance between the tissue region which is to be accessed by the catheter and the external body site at which the catheter is introduced. For example, where the catheter is introduced at a femoral site, and the target site is in the neck or head region, a relatively long proximal segment of up to 150 cm may be required. Catheters having substantially shorter lengths of about 50-70 cm would, or course, be suitable for applications in which the target tissue can be reached directly from a nearby external access site, such as a brachial artery for a target site in the head and neck.

With reference now to Figure 3, it is seen that the distal segment of the catheter is formed by a distal extension of tube 22 beyond the distal end of tube 20. As indicated above, the distal segment is formed of relatively flexible tubing, such as low-density polyethylene or silicone, and has a preferred final thickness of between about 0.05 to 0.1 mm (2-4 mils). The inner diameter of distal segment is preferably 0.05 to 0.12 mm (2-5 mils), and more preferably 0.07 to 0.1 mm (3-4 mils) larger than the associated guide wire. As noted above, this clearance acts to prevent buckling in the segment, by providing inner columnar support within the segment. At the same time, at least about a 0.05 to 0.12 mm (2-5 mil) clearance is necessary to accommodate tube distortion which occurs on tracking over a bend in the wire. That is, the 0.05 to 0.12 mm (2-5 mil) clearance prevents the tube from pinching against the wire in the region of the bend. (The bent tube assumes an oval cross-sectional shape which draws the sides of the tube toward the wire in the plane of the bend.)

A particular catheter construction which will be described herein is designed for use with an 0.45 mm (18 mil) guide wire with a tapered distal end encased in a uniform-diameter coil. The catheter is formed of a polypropylene inner tubing having a final wall thickness (in the finished catheter construction) of about 0.07 mm (3 mils), and a low-density polyethylene outer tubing also having a final wall thickness also about 0.07 mm (3 mils). The inner diameter of the catheter is between about 0.52 to 0.54 mm (21-22 mils) along its entire length, and the proximal and distal segments have lengths of about 100 and 10 cm, respectively.

Completing the description of the catheter, the free end of the proximal segment is attached to a fitting 24, such a standard syringe fitting, for use in connecting a syringe to the catheter for fluid injection. At the distal end of the catheter, a radio-opaque band 25 (Figure 9), such as a gold or platinum band, serves as a marker for following the position of the catheter radiographically.

The catheter can be formed by extruding an inner tubing of the appropriate material and wall thickness, according to conventional methods. The extruded tubing is then encased by conventional methods, such as heat shrinking, in an outer tubing which has a distal end portion which extends beyond the inner tube. The resulting catheter can be trimmed at one or both ends to produce the desired length proximal and distal segments. The catheter can also be formed by co-extrusion, according to known techniques. Here the two tubing materials are coextruded to form the proximal segment, with only the more flexible tubing material being extruded during the final (or initial) extrusion phase when the distal segment is being formed. In still another method, a relatively stiff tube suitable for the proximal segment of the catheter is placed tightly over a mandrel, and the mandrel and tube then coated, for example, by dipping or spraying with a polymer material, such as silicone which is suitable for forming the flexible distal segment. Alternatively, the catheter can be formed by methods in which the flexible distal tube is an extension of the inner, rather than outer, of the coaxial tubes forming the proximal segment.

In addition, it will be recognized that the coaxial arrangement of the catheter tubes, which provides a convenient way to link the relatively stiff tube in the proximal segment to the relatively flexible tube in the distal segment, does not require that the two tubes overlap along the entire proximal segment. That is, the area of overlap needed to join the two segments could be a relatively short portion of the proximal segment. Alternatively, if the materials forming the two catheter segments are heat or solvent fusable, the two segments can be joined end-to-end without tube overlapping.

Following tube construction, the proximal end of the coaxial tube is attached to fitting 24. Band 25 is attached by conventional methods to the end region of the distal segment.

In an embodiment of catheter in accordance with the present invention the above-described two-segment catheter construction is modified. This modified construction involves the catheter having one or more intermediate segments having flexibilities which are intermediate between those of the proximal and distal ends of the catheter. The purpose of the intermediate segment(s) is to provide greater column strength in the distal portion of the catheter which is moved along the tortuous path, but greater flexibility than is provided by the proximal segment of the catheter. The catheter segments can be formed, by extension of the above constructional principles, of three, two, and one tubing layers, respectively. In this modified embodiment, since the intermediate segment is used in tracking the wire along its tortuous path, it is considered as part of the distal segment. That is, the distal segment can be thought of as including a proximal portion (the intermediate segment) and a distal portion (the most flexible end segment). The modified construction of this embodiment provides a catheter that is particularly suited for accessing specific types of target area, such as for accessing a target site along a tortuous path which is as much as 25-30cm in length. In a preferred form of this modified construction, the distal portion of the distal segment, as well as the intermediate segment(s), are each between about 5-15cm long and together comprise between about 10%-40% of the total catheter length.

In the embodiments of catheter discussed above the flexibility of the catheter is step-wise variable. Alternatively the flexibility along the catheter, or selected portions thereof, may instead be made continuously variable. As an example, the distal segment may be continuously more flexible on proceeding toward the free end of the segment. This feature would be advantageous in a relatively long distal segment, especially where the greatest amount of flexibility is required deep within a target tissue.

### B. Guide Wire

Wire 14 is used for guiding the thin-wall catheter to the target site via a tissue vasculature or duct, and more specifically via a small-diameter tortuous vessel path at least about 20cm in length, and typically 20-40cm. The guide wire is a flexible torqueable guide wire having a total length between about 50-300cm, and a maximum diameter of the wire between about 0.2 to 1mm (8-40 mils). According to an important feature of the invention, the wire includes a region 26 of continuously diminishing diameter or taper, and which has a total length of at least about 20 cm and up to 50 cm or more, for movement through the small-diameter vessel path. The tapered region is typically located at the distal end region of the wire, as illustrated in Figure 1, but may also be flanked at either end by constant-diameter segments of the wire.

Figure 5 shows an expanded view of the tapered end region of the guide wire, and an associated end-region wire coil 32. As seen, the tapered region contains a continuous and linearly diminishing diameter extending from an upstream region indicated by arrow 28 and the distal tip of the wire. The portion proximal to region 26 is typically between about 50-150 cm long and terminates, at the proximal end of the wire, in a knob or removable handle 30 (Figure 1) which can be used to torque the wire manually to guide the wire to the tissue site, as will be considered below.

As indicated, the wire has a maximum diameter, upstream of the taper, of between about 0.2 to 1 mm (8-40 mils), and typically between about 0.2 to 0.4 mm (8-16 mils), and tapers to a minimum diameter typically between about 0.025 to 0.12 mm (1-5 mils), and preferably between about 0.025 to 0.25 mm (1-10 mils), at the distal tip. The total reduction in diameter, over the length of the tapered region, is typically between about 0.12 to 0.6 mm (5-25 mils). It will be appreciated that the scale of the drawing greatly exaggerates changes in the wire thickness.

Coil 32 is a soft, flexible coil which can be formed conventionally, e.g., as a winding of thin platinum wire. The coil is typically about 1-20 cm in length and preferably encases at least the distal half of the tapered region in the wire, terminating adjacent the end of the wire itself. The coil may have a fixed-dimension inner diameter, as shown, or a tapered diameter in the guide wire. Attachment of the coil to the wire is preferably by three solder or weld joints, including a proximal joint 33a, a rounded distal joint 33b, and an intermediate joint 33c which is preferably about 1-3 cm from the distal end of the wire and coil. The intermediate joint serves to transmit torque in the wire to the coil, to cause the end region of the coil (and wire) to bend slightly at the solder joint, to allow the wire to be guided in a selected direction in a vessel network, by torquing the proximal end of the wire.

In addition to providing a mechanism for wire bending near the wire tip, the coil also gives the covered region of the wire increased column strength (in the axial direction), and reduces the chance of irreversible bending in the tip of the wire. At the same time, the combined flexibility of the wire and coil is compatible with a series of sharp bends, as the wire is moved through a tortuous tissue pathway. The rounded joint at the end of the wire acts to shield vessel walls from the sharp end of the wire.

Figure 6 shows the tapered distal end region 34 of a guide wire 35 constructed according to another embodiment of the invention. The continuously diminishing diameter region is characterized by a taper which is slightly concave with respect to a linear taper (shown in dotted lines in the figure). This embodiment provides greater flexibility, but less strength and torque in the proximal portion of the tapered region than does the linear-taper wire of Figure 5. Although not shown, the wire may be equipped with a fixed-diameter or tapered flexible coil, such as coil 32 described with respect to Figure 5.

Figure 7 shows the tapered distal end region 36 of a guide wire 37 constructed according to another embodiment of the invention. The continuously diminishing diameter region is characterized by a taper which is somewhat convex with respect with the linear taper (also indicated here by dotted lines). This embodiment provides greater strength and torqueability, but less flexibility in the proximal portion of the tapered region than the linear-taper wire of Figure 5. As above, this wire may be equipped with a flexible coil.

Additional types of non-linear tapers are also contemplated by the invention. For example, the continuously tapered region of the wire may include both convex and concave tapers, typically in a configuration in which the proximal portion of the tapered region has a convex taper, and the remaining distal portion, concave taper. The concave tapered portion may be covered by a flexible coil for added column strength and resistance to irreversible bending.

The guide wire may be formed from flexible metal wire having a length typically between about 50-300 cm and a selected diameter between about 0.2 to 1 mm (8-40 mils). Stainless steel wire of this type is commercially available, such as from Wytech and National Standard.

The tapered region of the metal wire may be formed by wire grinding, drawing, or etching techniques. Wire grinding is preferably carried out using a conventional grinding apparatus in which a rotating workpiece (the wire) is moved axially on a shiftable carriage past a grinding wheel, with the radial distance between the edge of the grinding wheel and the center of the wire axis being continuously varied (reduced) as the workpiece is advanced axially, to produce a continuously variable depth of cut. The mechanism used to vary the radial distance between the wire and grinding wheel may be a mechanical cam device which operatively couples the motion of the carriage to the frame of the grinding machine on which the grinding wheel is mounted. Alternatively, the mechanism may be an electronically controlled device whose movement toward and away from the grinding wheel is determined by the axial position of the workpiece. The grinding apparatus is modified for use in fine-wire grinding as follows: The machine has a small-diameter chuck for holding the wire for rotation and axial movement, and includes structure to stabilize the rotating wire.

The advantages of the grinding method are accurate control over the depth of cut along the wire, and the ability to produce the continuous taper at any region along the length of the wire.

Etching techniques for etching stainless steel substrates are known. In forming the guide wire of the invention, the region or regions to be tapered are submerged in an chemical etching bath. The wire is gradually withdrawn from the bath at a rate which exposes submerged portions of the wire to greater etching and therefore greater reduction in wire diameter. The rate of wire removal can be adjusted to produce linear, concave, or convex tapers.

The etching method has the advantage that many wires can be processed in single batch. Also, complex machining and or wire processing devices are avoided.

Wire drawing, in which a heated wire is drawn under tension to a desired diameter, can also be used to produce long regions of continuous taper in the wire. In this method, the selected region of wire is heated, for example within a firing oven, and drawn out at a preselected tension when a selected wire temperature is reached. By careful monitoring of wire temperature and temperature uniformity in the heated wire region, and rate of wire drawing, accurate continuous taper can be produced.

Other wire materials which have suitable flexibility and torqueability may also be used in forming the tapered guide wire. Studies by the inventors indicate that small-diameter glass wires (rods or tubes) have flexibility and torqueability properties which are suitable for guide wire use, at wire diameters of between about 0.2 to 1 mm (8-40 mils). In fact, fiberglass wires have the advantage of good torque transmission, even when the wire is extensively looped or bent.

Extended regions of continuous taper in a fiberglass wire can be produced by drawing techniques, such as those described above for metal wires. The tapered wire may be covered with a polymer sheath or the like to increase bending strength and to reduce the hazard of broken wire ends in a tissue accessing application.

Figure 8 shows a tapered composite wire 38 formed according to another embodiment of the invention. The wire is constructed by laying down an bundle of long parallel fibers, such as carbon fibers 39, in a small-diameter array. The bundle is then infused with a suitable resin, such as epoxy, to form the composite wire. The infusion may be carried out in a small-diameter wire mold. In this method the fibers in the fiber bundle in the tapered region of the wire terminate at various points along the length of the region, giving progressively fewer fibers on progressing toward the smaller diameter end of the taper, as indicated in Figure 8. Alternatively, a constant diameter rod with a uniform bundle of fibers may be machined, as described above, to form the continuously diminishing region of the wire.

### II. Operation

The method of inserting the catheter into a tissue region which is reached by a tortuous path will be described now with reference to Figure 9. The figure shows a region of soft tissue 40, such as brain tissue, containing a target site 42 which is to be accessed by the catheter. Initially, the guide wire, such as wire 14, is fed from a vascular access region adjacent the target tissue into a tissue-supply vessel 44 which extends into the tissue. In the present example, the tortuous path to the target site involves vessel 44, a vessel 46 which branches off vessel 44 at more than a right angle, and branch vessel 48 and 50, which each branch off the preceeding vessel as shown. The path shown involves (a) a number of bends, some of which may be 90 degrees or more, (b) small vessels, typically with lumen diameters of less than about 3 mm, and (c) a total path length within the target tissue of at least about 5 cm, typically between about 10-15 cm, and as much as 25 cm.

A path having these characteristics is defined herein as a tortuous path, and is also characterized as being accessible by a guidewire 0.46 mm (18 mil) or smaller of the type described above, but being too delicate and/or tortuous for accessing by a significantly larger-diameter guidewire.

In operation, the catheter device is threaded as a unit from an external access site through the vasculature to a region adjacent, but not into the tortuous path region of the target tissue. This is done, in the usual case where the catheter must pass through the cardiac aorta, by first placing a relatively large diameter guiding catheter (e.g., about 1 mm (40 mils) inner diameter) from the access site through the aorta and toward the target site. The present catheter and guidewire are then threaded through the guiding catheter past the aorta, where large-vessel diameters and high blood flow volumes make it difficult or impossible to control the movement and position of the catheter. Once beyond the guiding catheter, the present catheter and guide wire can be advanced as a unit toward the target site. In general, the path from the access site to the region adjacent the tissue is easily accessible, in that sharp bends, small-lumen vessels, and or soft tissue structure are not encountered. Typically, when the tortuous path tissue region is reached, and particularly where sharp bends in the path are encountered, the wire is advanced ahead of the catheter. This is done by advancing the wire axially within the catheter and at the same time torquing the wire to orient the bent tip of the wire in the direction of desired wire movement. After the wire has been so advanced, the catheter is then advanced over the wire until the catheter end is close to the wire end and this procedure is repeated until the wire and catheter have been fully advanced through the small-diameter tissue vessel region to the target tissue site. Alternatively, the wire and catheter can be advanced as a unit through the small-vessel region. However, this approach allows less maneuverability since the flexibility of the wire is reduced when it is ensheathed by the catheter, and is therefore likely to be used only in advancing the wire and catheter along sections of the path which do not contain sharp bends.

Several features of catheter device contribute to the ability to guide the catheter to reach soft tissue sites which have heretofore been inaccessible because of the small diameters and sharp bends in the vessel pathways leading to the site. The advantages inherent in the novel guide wire construction can be appreciated with reference to Figures 10-13, which are enlarged views of the tissue region indicated by dash-dot line in Figure 9. Figure 10 illustrates wire and catheter deformations which can occur when a catheter device having a variable-step guide wire 58 of the type known in the prior art and a catheter 60 is guided through a small-diameter tortuous path tissue region. The portion of the wire shown here has two variable-step regions-- a first region 61, and a second region 62. The truncation in vessel 46 indicates that the two tapered regions may in fact be separated by a relatively long segment of constant-diameter wire.

As seen in the figure, when a sharp bend in the path occurs at the distal end of a taper, the wire tends to bend most sharply at the distal end of the taper, where the wire assumes a smaller, constant-diameter thickness. If the bend in the wire at this taper interface is sufficiently sharp, as tends to occur at a sharp bend in the vessel path, the thin-wall catheter may buckle, as shown in the figure. It can be appreciated with reference to Figures 10 and 11 that sharp catheter bending deforms the cross section of the catheter (indicated at 60) in a way that substantially limits sliding movement of wire within the catheter. This is especially true for wire movement in a downstream direction, because of the increased diameter of the wire at the taper (seen head on in Figure 11). In addition, the sharp turn in the wire decreases torque efficiency, and increases the risk that the wire will break on torquing, especially because of the torquing strain which tends to occur at the distal end of each taper region.

The problem of catheter buckling is further compounded if two variable-step regions of the guide wire coincide with two sharp turns in the vessel path, as illustrated in Figure 10. Here catheter buckling at two spaced location makes sliding movement within the catheter virtually impossible, either in a downstream or upstream direction. This situation will occur whenever the spacing between two turns in a tortuous tissue path coincides with the distance between two step regions in the guide wire, and is frequently encountered in catheter placement operations involving small-vessel regions. When the problem does occur, the radiologist performing the catheter procedure must either attempt to advance the wire and catheter as a unit toward the selected tissue site, or withdraw the wire and catheter and attempt to place the catheter with a guide wire having a different step-region spacing.

Figure 12 shows a catheter placement operation through the same tissue region, using catheter device 12 having a continuously variable guide wire 14. Since the wire does not contain transitions zones with appreciable changes in bending modulus, the sharp angle bending which can occur in the transitions zones of variable-step wires seen in Figure 10 are avoided. That is, the sharpest angles which are permitted in the present guide wire are substantially the same as are permitted in the constant-diameter regions of a variable-step wire. Accordingly, the cross-sectional distortion in the catheter and the tendency to buckle are significantly reduced, as indicated in Figures 12 and 13. Furthermore, even where the catheter does become somewhat pinched (elliptical) in a bend region, the change in wire diameter is only very slight in moving upstream, allowing the wire to be advanced readily through the catheter in a catheter-placement operation. It will be appreciated that the above considerations relating to wire bending would also apply if the distal end portion of the tapered region is covered by a flexible coil such as coil 32. Figure 12 also shows a taper in the flexible distal end segment 18 of catheter 12.

Torque transmission in the continuously tapered wire is also enhanced, both because sharp bends in the wire and torque losses across variable-step taper zones are minimized. This allows better maneuverability of the wire and also reduces the risk of the wire breaking at a sharp-turn transition zone.

In addition to the above advantages provided by the guide wire, three features inherent in the catheter contribute to the ability to advance the catheter along the guide wire in a small-diameter tortuous pathway. The first is the flexibility of the distal segment, which allows the catheter to follow or track the bend without a significant increase in the force required to advance the catheter. That is, the flexible distal segment catheter is able to move relatively freely over the bend in the wire in the direction of catheter advance. The second feature is the relatively short length (e.g., 5-15 cm) of the flexible distal segment, and more particularly, the distance along the distal segment from the end of the proximal segment to the bend in the guidewire. The shorter length means greater column strength (strength in resisting axial stress), with reduced tendency to buckle. As described above, to cope with longer path lengths, the distal segment of the catheter of the present invention includes one or more intermediate segments of intermediate stiffness to provide greater column strength in the catheter along its distal end region. The third feature is the small clearance between the guide wire and inner wall of the distal segment, which acts to provide columnar support for the catheter segment, as indicated above. As noted above, the 0.05 to 0.125 mm (2-5 mil) clearance is sufficient to accommodate the distortion in the distal segment which occurs at bends in the guide wire, without causing appreciable frictional contact between the tube and guidewire, as the tube tracks over the bend. Buckling in the relatively long proximal segment is prevented by the stiffness in the segment combined with the column support provided by the guide wire.

After the catheter has been moved to the target site, the guide wire is withdrawn to allow a fluid material to be injected into the site. The injected material may include: (1) radio-opaque agents, for viewing blood vessel anatomy and blood flow characteristics in the target region, (2) vaso-occlusive agents, such as a suspension of collagen fibers, which can be used to produce small-artery vaso-occlusion in the tissue region supplied by the target vessel; and (3) pharmacological agents, such as anti-tumor drugs, which are effective against identified disease states at the target site.

The novel catheter device includes a guide wire whose tapered distal end allows for good manueverability and torque transmission in a small-diameter tortuous pathway, without sharp wire bending. Accordingly, problems of catheter buckling and inability to advance or withdraw the wire through the catheter are minimized. The catheter construction described herein allows for tracking along a tortuous path over a guide wire without buckling by virtue of (1) the relatively flexible distal portion which tracks over the wire along the tortuous path, (2) the relatively stiff proximal portion which extends over a major portion of the catheter length, and (3) the guidewire clearance within the catheter which provides column support, particularly within the distal segment, while still accommodating distortion in the segment in the region of bending.

It is now possible, with this combination of advantages, to access target sites along small-diameter tortuous pathways in soft tissue, such as deep brain sites, which have heretofore been inaccessible to catheters. This allows for the delivery of diagnostic, therapeutic or vaso-occlusive agents to deep tissue sites, without need for invasive surgery, and with more precise tissue target.

The catheter and guide wire components of the catheter device are both readily constructed by the various methods described above.

## Claims

1. A catheter (12) for use with a guidewire (14), the catheter being guidable from an external body access site to and into a soft internal brain or liver tissue, the catheter comprising:
an elongate tubular member having proximal and distal ends and an inner lumen (13) extending between the proximal and distal ends, said member being composed of:
a relatively stiff proximal segment (16) dimensioned to track the guidewire from the access site to a region adjacent the internal tissue;
a relatively flexible distal segment (18) constructed and dimensioned to track the guidewire from said region to a target site within the internal tissue along the entire length of a tortuous path within the internal tissue of at least about 5 cm, through vessels of less than about 3 mm lumen inner diameter and around a plurality of sharp bends some of which may be of 90° or more, by means of an axially directed force applied to the distal segment through the proximal segment, the distal segment (18) having both a proximal portion and a distal portion and said proximal portion including one or more intermediate segments having lesser flexibility than the distal portion of the distal segment (18) and greater flexibility than the proximal segment (16).

2. A catheter as claimed in claim 1, wherein said distal portion and said intermediate segment(s) are each between 5-15 cm long.

3. A catheter as claimed in claim 1 or claim 2, wherein said distal portion and said intermediate segment(s) together comprise 10% to 40% of the total length of the catheter.

4. A catheter as claimed in any one of the preceding claims, comprising but a single said intermediate segment.

5. A catheter as claimed in any one of the preceding claims, wherein the flexibility along selected portions of the catheter varies continuously.

6. A catheter as claimed in claim 5, wherein the distal segment (18) is continuously more flexible on proceeding towards the free distal end of the segment.

7. A catheter as claimed in any one of claims 1 to 4, wherein the flexibility of the catheter is step-wise varied.

8. A catheter as claimed in any one of the preceding claims, wherein said tubular member comprises a plurality of tubes.

9. A catheter as claimed in claim 8, wherein the plurality of tubes are of different flexibilities and include an overlap, the combined wall thickness of the tubes at the overlap(s) not exceeding 0.25 mm (10 mils).

10. A catheter as claimed in claim 8 when dependent on claim 4, wherein said elongate tubular member comprises three tubes of differing length and which are coaxially overlapped, different areas of overlap of said three tubes forming different ones of said proximal segment (16), said distal portion and said intermediate segment(s).

11. A catheter as claimed in claim 9 or claim 10, wherein the outermost tube (22) extends substantially the entire length of the catheter.

12. A catheter as claimed in any one of claims 8 to 11, wherein said ones of said tubes (20, 22) are formed of a polymeric material being substantially polypropylene, high density polyethylene or low density polyethylene.

13. A catheter as claimed in any one of claims 8 to 12, wherein the tubes (20, 22) each have a wall thickness of between 0.05 and 0.1 mm (2 and 4 mils).

14. A catheter as claimed in any one of the preceding claims, wherein said distal segment (18) is of 5-15 cm in length measured from the distal end.

15. A catheter as claimed in any one of the preceding claims, wherein the proximal segment (16) has a total length of between about 60-150 cm and the distal segment (18) has a total length of between about 10-15 cm.

16. A catheter as claimed in any one of the preceding claims, wherein the proximal segment (16) makes up between 70%-95% of the total length of the tubular member and the distal segment (18) makes up the remaining 5%-30%.

17. A catheter as claimed in any one of the preceding claims, wherein the lumen (13) of said tubular member has a substantially uniform inner diameter along said distal segment (18).

18. A catheter as claimed in any one of the preceding claims, further comprising the guidewire (14), said guidewire being slidably receivable within said inner lumen (13).

19. A catheter as claimed in claim 18, wherein said guidewire (14) is flexible, torqueable wire having a length between 50-300 cm between its proximal and distal ends.

20. A catheter as claimed in claim 18 or claim 19, wherein said guidewire includes a distal end region (26) having a continuously diminishing diameter on progressing towards the distal end of the wire (14).

21. A catheter as claimed in claim 20, wherein the distal end region of continuously diminishing diameter is at least 10 cm in length.

22. A catheter as claimed in any of claims 18 to 21, wherein the guidewire (14) has a flexible coil (32) extending along and encasing a portion of a or the distal end region (26) of the guidewire.

23. A catheter as claimed in any of claims 18 to 22, wherein the clearance between a or the distal end region of said guidewire (14) and said distal segment (18) of the catheter is, in use, between 0.05-0.12 mm (2-5 mils).

24. A catheter as claimed in any of claims 18 to 23, wherein said guidewire (14) has a specified diameter and said distal segment (18) of said catheter has an inner diameter which is between 0.05 to 0.12 mm (2-5 mils) larger than said specified diameter of the guidewire (14).

25. A catheter as claimed in claim 24, wherein said distal segment (18) of said catheter has an inner diameter which is between 0.07 to 0.1 mm (3-4 mils) larger than said specified diameter of the guidewire (14).

26. A catheter as claimed in any of claims 18 to 25, wherein the clearance between a proximal end region of said guidewire (14) and said proximal segment (16) of the catheter is, in use, between 0.05 to 0.12 mm (2-5 mils)

## Patentansprüche

1. Katheter (12) zur Verwendung mit einem Führungsdraht (14), wobei der Katheter von einer externen Körpereintrittsstelle aus an ein internes Gehirn- oder Lebergewebe heran- und in dieses einführbar ist, umfassend
ein langgestrecktes rohrförmiges Teil mit einem proximalen und einem distalen Ende und einem sich zwischen dem proximalen und dem distalen Ende erstreckenden inneren Lumen (13), wobei das Teil besteht aus:
einem relativ steifen proximalen Segment (16) mit zum Verfolgen des Führungsdrahtes von der Eintrittsstelle zu einem dem internen Gewebe benachbarten Bereich geeigneten Abmessungen,
einem relativ flexiblen distalen Segment (18) mit einem Aufbau und Abmessungen zum Verfolgen des Führungsdrahtes von dem Bereich zu einer Zielstelle innerhalb des internen Gewebes längs der gesamten Länge eines gewundenen Weges innerhalb des internen Gewebes von mindestens etwa 5 cm durch Gefäße von weniger als etwa 3 mm innerem Lumendurchmesser und um eine Mehrzahl scharfer Biegungen, von denen einige um einen Winkel von 90° oder mehr führen können, mittels einer durch das proximale Segment auf das distale Segment ausgeübten, axial gerichteten Kraft, wobei das distale Segment (18) sowohl einen proximalen Bereich als auch einen distalen Bereich aufweist und der proximale Bereich ein oder mehrere Zwischensegmente mit einer geringeren Flexibilität als der distale Bereich des distalen Segments (18) und einer größeren Flexibilität als das proximale Segment (16) aufweist.

2. Katheter nach Anspruch 1, bei dem der distale Bereich und das (die) Zwischensegment(e) jeweils zwischen 5-15 cm lang sind.

3. Katheter nach Anspruch 1 oder Anspruch 2, bei dem der distale Bereich und das oder die Zwischensegment(e) zusammen 10 % bis 40 % der Gesamtlänge des Katheters ausmachen.

4. Katheter nach einem der vorhergehenden Ansprüche, der nur ein einziges solches Zwischensegment aufweist.

5. Katheter nach einem der vorhergehenden Ansprüche, bei dem die Flexibilität längs ausgewählten Bereichen des Katheters kontinuierlich veränderlich ist.

6. Katheter nach Anspruch 5, bei dem das distale Segment (18) zum freien distalen Ende des Segments hin fortschreitend kontinuierlich flexibler ist.

7. Katheter nach einem der Ansprüche 1 bis 4, bei dem die Flexibilität des Katheters stufenweise veränderlich ist.

8. Katheter nach einem der vorhergehenden Ansprüche, bei dem das rohrförmige Teil eine Mehrzahl von Rohren aufweist.

9. Katheter nach Anspruch 8, bei dem die Mehrzahl von Rohren in unterschiedlichen Flexibilitäten ausgebildet ist und eine Überlappung aufweist, wobei die kombinierte Wandstärke der Rohre an der (den) Überlappung(en) 0,25 mm (10 mils) nicht überschreitet.

10. Katheter nach Anspruch 8 in Abhängigkeit von Anspruch 4, bei dem das langgestreckte rohrförmige Teil drei Rohre von unterschiedlicher Länge, die sich koaxial überlappen, aufweist, wobei unterschiedliche Überlappungsbereiche der drei Rohre die unterschiedlichen Abschnitte: proximales Segment (16), distaler Bereich und Zwischensegment(e) bilden.

11. Katheter nach Anspruch 9 oder 10, bei dem sich das äußerste Rohr (22) im wesentlichen über die gesamte Länge des Katheters erstreckt.

12. Katheter nach einem der Ansprüche 8 bis 11, bei dem die Rohre (20, 22) aus einem polymeren Material gebildet sind, das im wesentlichen Polypropylen, Polyäthylen hoher Dichte (HDPE) oder Polyäthylen niedriger Dichte (LDPE) ist.

13. Katheter nach einem der Ansprüche 8 bis 12, bei dem die Rohre (20, 22) jeweils eine Wandstärke zwischen 0,05 und 0,1 mm (2 und 4 mils) aufweisen.

14. Katheter nach einem der vorhergehenden Ansprüche, bei dem das distale Segment (18) eine Länge von 5-15 cm, gemessen vom distalen Ende, aufweist.

15. Katheter nach einem der vorhergehenden Ansprüche, bei dem das proximale Segment (16) eine Gesamtlänge zwischen etwa 60-150 cm und das distale Segment (18) eine Gesamtlänge zwischen etwa 10-15 cm aufweist.

16. Katheter nach einem der vorhergehenden Ansprüche, bei dem das proximale Segment (16) zwischen 70 % bis 95 % der Gesamtlänge des rohrförmigen Teils und das distale Segment (18) die verbleibenden 5%-30% ausmacht.

17. Katheter nach einem der vorhergehenden Ansprüche, bei dem das Lumen (13) des rohrförmigen Teils längs dem distalen Segment (18) einen im wesentlichen gleichbleibenden Innendurchmesser aufweist.

18. Katheter nach einem der vorhergehenden Ansprüche, der ferner den Führungsdraht (14) aufweist, wobei der Führungsdraht gleitend in das innere Lumen (13) aufnehmbar ist.

19. Katheter nach Anspruch 18, bei dem der Führungsdraht (14) ein flexibler, tordierbarer Draht ist, der zwischen seinem proximalen und distalen Ende eine Länge zwischen 50-300 cm aufweist.

20. Katheter nach Anspruch 18 oder Anspruch 19, bei dem der Führungsdraht einen distalen Endbereich (26) aufweist, der zum distalen Ende des Drahtes (14) hin fortschreitend einen kontinuierlich abnehmenden Durchmesser aufweist.

21. Katheter nach Anspruch 20, bei dem der distale Endbereich kontinuierlich abnehmenden Durchmessers eine Länge von mindestens 10 cm aufweist.

22. Katheter nach einem der Ansprüche 18 bis 21, bei dem der Führungsdraht (14) eine flexible Wendel (32) aufweist, die sich längs eines Bereichs eines oder des distalen Endbereichs (26) des Führungsdrahtes erstreckt und diesen umschließt.

23. Katheter nach einem der Ansprüche 18 bis 22, bei dem der Spielraum zwischen einem oder dem distalen Endbereich des Führungsdrahts (14) und dem distalen Segment (18) des Katheters in dessen Anwendung zwischen 0,05-0,12 mm (2-5 mils) beträgt.

24. Katheter nach einem der Ansprüche 18 bis 23, bei dem der Führungsdraht (14) einen vorgegebenen Durchmesser und das distale Segment (18) des Katheters einen Innendurchmesser aufweist, der zwischen 0,05 bis 0,12 mm (2-5 mils) größer ist als der vorgegebene Durchmesser des Führungsdrahtes (14).

25. Katheter nach Anspruch 24, bei dem das distale Segment (18) des Katheters einen Innendurchmesser aufweist, der zwischen 0,07 bis 0,1 mm (3-4 mils) größer ist als der vorgegebene Durchmesser des Führungsdrahtes (14).

26. Katheter nach einem der Ansprüche 18 bis 25, bei dem der Spielraum zwischen einem proximalen Endbereich des Führungsdrahtes (14) und dem proximalen Segment (16) des Katheters in der Anwendung zwischen 0,05 bis 0,12 mm (2-5 mils) beträgt.

## Revendications

1. Cathéter (12) destiné à être utilisé avec un fil de guidage (14), le cathéter état guidable d'un site d'accès corporel externe vers et dans un tissu mou interne cérébral ou hépatique, le cathéter comprenant :
un élément tubulaire allongé ayant des extrémités proximale et distale et une lumière interne (13) qui s'étend entre les extrémités proximale et distale, ledit élément état constitué par :
un segment proximal (16) relativement rigide dimensionné pour suivre le fil de guidage du site d'accès à une région adjacente au tissu interne ; et
un segment distal (18) relativement flexible conçu et dimensionné pour suivre le fil de guidage de ladite région à un site cible dans le tissu interne sur toute la longueur d'un passage tortueux dans le tissu interne d'au moins environ 5 cm, par des vaisseaux ayant une lumière de diamètre interne inférieur à environ 3 mm et en épousant une pluralité de coudes aigus dont certains peuvent avoir 90° ou plus, au moyen d'une force dirigée axialement, appliquée au segment distal par l'intermédiaire du segment proximal, le segment distal (18) ayant une partie proximale et une partie distale et ladite partie proximale comprenant un ou plusieurs segments intermédiaires ayant une flexibilité inférieure à la partie distale du segment distal (18) et une flexibilité supérieure au segment proximal (16).

2. Cathéter selon la revendication 1, dans lequel ladite partie distale et ledit ou lesdits segments intermédiaires ont chacun entre 5 et 15 cm de long.

3. Cathéter selon la revendication 1 ou la revendication 2, dans lequel ladite partie distale et ledit ou lesdits segments intermédiaires constituent ensemble 10 à 40 % de la longueur totale du cathéter.

4. Cathéter selon l'une quelconque des revendications précédentes, comprenant un seul segment intermédiaire.

5. Cathéter selon l'une quelconque des revendications précédentes, dans lequel la flexibilité le long de parties choisies du cathéter varie de manière continue.

6. Cathéter selon la revendication 5, dans lequel le segment distal (18) est continuellement plus flexible en direction de l'extrémité distale libre du segment.

7. Cathéter selon l'une quelconque des revendications 1 à 4, dans lequel la flexibilité du cathéter est modifiée par pas.

8. Cathéter selon l'une quelconque des revendications précédentes, dans lequel ledit élément tubulaire comprend une pluralité de tubes.

9. Cathéter selon la revendication 8, dans lequel la pluralité de tubes ont des flexibilités différentes et comprennent un chevauchement, l'épaisseur combinée des parois des tubes au niveau du ou des chevauchements ne dépassant pas 0,25 mm (10 millièmes de pouce).

10. Cathéter selon la revendication 8 dépendante de la revendication 4, dans lequel ledit élément tubulaire allongé comprend trois tubes de longueurs différentes et qui sont en chevauchement coaxial, différentes zones de chevauchement desdits trois tubes formant des zones différentes dudit segment proximal (16), de ladite partie distale et dudit ou desdits segments intermédiaires.

11. Cathéter selon la revendication 9 ou la revendication 10, dans lequel le tube externe (22) s'étend sensiblement sur toute la longueur du cathéter.

12. Cathéter selon l'une quelconque des revendications 8 à 11, dans lequel lesdits tubes (20, 22) sont en une matière polymère qui est essentiellement du polypropylène, du polyéthylène haute densité ou du polyéthylène basse densité.

13. Cathéter selon l'une quelconque des revendications 8 à 12, dans lequel les tubes (20, 22) ont chacun une épaisseur de paroi comprise entre environ 0,05 et 0,1 mm (2 et 4 millièmes de pouce).

14. Cathéter selon l'une quelconque des revendications précédentes, dans lequel ledit segment distal (18) a une longueur, mesurée depuis l'extrémité distale, de 5 à 15 cm.

15. Cathéter selon l'une quelconque des revendications précédentes dans lequel le segment proximal (16) a une longueur totale comprise entre environ 60 et 150 cm et le segment distal (18) a une longueur totale comprise entre environ 10 et 15 cm.

16. Cathéter selon l'une quelconque des revendications précédentes, dans lequel le segment proximal (16) constitue entre 70 % et 95 % de la longueur totale de l'élément tubulaire et le segment distal (18) constitue les 5 % à 30 % restants.

17. Cathéter selon l'une quelconque des revendications précédentes, dans lequel la lumière (13) dudit élément tubulaire a un diamètre interne sensiblement uniforme le long dudit segment distal (18).

18. Cathéter selon l'une quelconque des revendications précédentes, comprenant en outre le fil de guidage (14), ledit fil de guidage pouvant être reçu à glissement à l'intérieur de ladite lumière interne (13).

19. Cathéter selon la revendication 18, dans lequel ledit fil de guidage (14) est un fil flexible, déformable, ayant une longueur comprise entre 50 et 300 cm entre ses extrémités proximale et distale.

20. Cathéter selon la revendication 18 ou la revendication 19, dans lequel ledit fil de guidage comprend une région d'extrémité distale (26) ayant un diamètre qui diminue de manière continue en direction de l'extrémité distale du fil (14).

21. Cathéter selon la revendication 20, dans lequel la région d'extrémité distale de diamètre qui diminue de manière continue a au moins 10 cm de long.

22. Cathéter selon l'une quelconque des revendications 18 à 21, dans lequel le fil de guidage (14) a une spirale flexible (32) s'étendant le long d'une partie d'une ou de la région d'extrémité distale (26) du fil de guidage et l'entourant.

23. Cathéter selon l'une quelconque des revendications 18 à 22, dans lequel l'écartement entre une ou la région d'extrémité distale dudit fil de guidage (14) et ledit segment distal (18) du cathéter est compris, au cours de l'utilisation, entre 0,05 et 0,12 mm (2 à 5 millièmes de pouce).

24. Cathéter selon l'une quelconque des revendications 18 à 23, dans lequel ledit fil de guidage (14) a un diamètre spécifié et ledit segment distal (18) dudit cathéter a un diamètre interne qui est supérieur de 0,05 à 0,12 mm (2 à 5 millièmes de pouce) audit diamètre spécifié du fil de guidage (14).

25. Cathéter selon la revendication 24, dans lequel ledit segment distal (18) dudit cathéter a un diamètre interne qui est supérieur de 0,07 à 0,1 mm (3 à 4 millièmes de pouce) audit diamètre spécifié du fil de guidage (14).

26. Cathéter selon l'une quelconque des revendications 18 à 25, dans lequel l'écartement entre une région d'extrémité proximale dudit fil de guidage (14) et ledit segment proximal (16) du cathéter est compris, au cours de l'utilisation, entre 0,05 et 0,12 mm (2 à 5 millièmes de pouce).
